(19)

Europäisches
Patentamt
European
Patent Office
Office européen
des brevets

(11) **EP 4 589 603 A1**

(12) **EUROPEAN PATENT APPLICATION**

(43) Date of publication:
**23.07.2025 Bulletin 2025/30**

(21) Application number: **25152263.7**

(22) Date of filing: **16.01.2025**

(51) International Patent Classification (IPC):
**G16H 50/20** (2018.01)

(52) Cooperative Patent Classification (CPC):
**G16H 50/20; G16H 20/40; G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.01.2024 CN 202410067328**

(71) Applicant: **ChinaBridge (Shenzen) Medical
Technology Co., Ltd.
Shenzhen, Guangdong 518102 (CN)**

(72) Inventor: **LI, Yijiang
Shenzhen Guangdong, 518102 (CN)**

(74) Representative: **Pfenning, Meinig & Partner mbB
Patent- und Rechtsanwälte
Joachimsthaler Straße 10-12
10719 Berlin (DE)**

(54) **A METHOD, DEVICE, EQUIPMENT, AND MEDIUM FOR EVALUATING PATIENT ECMO OPERATION STATUS**

(57) A method for evaluating a patient's extracorporeal membrane oxygenation (ECMO) operation status includes: collecting the patient's oxygen saturation data, lactate data, and oxygen partial pressure data or mean arterial pressure data; based on oxygen partial pressure data, obtaining the oxygen partial pressure classification value through a pre-trained oxygen partial pressure classifier, or based on the mean arterial pressure data, obtaining the mean arterial pressure classification value through a pre-trained mean arterial pressure classifier; based on blood oxygen saturation data, obtaining the blood oxygen saturation classification value through the pre-trained blood oxygen saturation classifier; based on the lactate data, obtaining the lactate level classification value through the pre-trained lactate level classifier; determining the patient's ECMO operation score based on the blood oxygen saturation classification value, lactate level classification value, and oxygen partial pressure classification value or mean arterial pressure classification value.

Figure 1

EP 4 589 603 A1

**Description**

**Technical Field**

The present disclosure relates to the technical field of medical evaluation, and in particular to a method, device, equipment, and medium for evaluating a patient's extracorporeal membrane oxygenation (ECMO) operation status.

**Background**

According to current clinical feedback, when intensive care unit (ICU) patients currently utilize ECMO equipment, the ECMO equipment does not provide the ability to obtain physiological parameter data from other monitoring equipment (patient monitors, blood gas analyzers, etc.) while the ECMO equipment is running. In some scenarios, when ICU medical staff need to conduct data integration and analysis, there can be limitations in doing so, for example, due to differences in the personal experience of doctors and nurses. In some scenarios, there is a lack of standards for patient assessment, judgment, and corresponding treatment. Thus, there is individual subjectivity due to the a lack of objective standards.

**Summary**

Embodiments of the present disclosure provide a method, device, equipment and medium for evaluating a patient's ECMO operation status, aiming to solve the problem in the existing technology that it is difficult to objectively evaluate the physiological status of patients using ECMO equipment during operation. Programmed prompts can be used to evaluate the patient's ECMO operation status. Provide users with handling suggestions to reduce operating errors.
In a first aspect, examples of the present disclosure provide a method for assessing patient ECMO operation, which includes:

    Collect the patient's oxygen saturation data, lactate data, partial pressure of oxygen or mean arterial pressure data;
    Obtain the oxygen partial pressure classification value through a pre-trained oxygen partial pressure classifier based on the oxygen partial pressure data, or obtain the mean arterial pressure classification value through a pre-trained mean arterial pressure classifier based on the mean arterial pressure data;
    Obtain the blood oxygen saturation classification value through a pre-trained blood oxygen saturation classifier based on the blood oxygen saturation data;
    Obtain a lactate level classification value through a pre-trained lactate level classifier based on the lactate data;
    Determine the patient's ECMO performance score based on the blood oxygen saturation classification value, the lactate level classification value and the oxygen partial pressure classification value or the mean arterial pressure classification value.

A further technical solution is to obtain the oxygen partial pressure classification value through a pre-trained oxygen partial pressure classifier based on the oxygen partial pressure data, including:

    Input the oxygen partial pressure data into the oxygen partial pressure classifier, and receive the oxygen partial pressure marker value output by the oxygen partial pressure classifier;
    Determine the oxygen partial pressure classification value based on the oxygen partial pressure marker value;

Based on the mean arterial pressure data, the mean arterial pressure classification value is obtained through a pre-trained mean arterial pressure classifier, including:

    Input the mean arterial pressure data into the mean arterial pressure classifier, and receive the mean arterial pressure marker value output by the mean arterial pressure classifier;
    Determine the mean arterial pressure classification value based on the mean arterial pressure marker value;

The blood oxygen saturation classification value is obtained through a pre-trained blood oxygen saturation classifier based on the blood oxygen saturation data, including:

    Input the blood oxygen saturation data into the blood oxygen saturation classifier, and receive the blood oxygen saturation marker value output by the blood oxygen saturation classifier;
    Determine the blood oxygen saturation classification value based on the blood oxygen saturation marker value;

Based on the lactate data, the lactate level classification value is obtained through a pre-trained lactate level classifier, including:

Input the lactate data into the lactate level classifier, and receiving a lactate level marker value output by the lactate level classifier;

Determine the lactate level classification value based on the lactate level marker value.

A further technical solution is to determine the oxygen partial pressure classification value based on the oxygen partial pressure marker value, including:Determine the oxygen partial pressure classification value corresponding to the oxygen partial pressure marker value based on the preset oxygen partial pressure-classification value mapping relationship;

The determination of the mean arterial pressure classification value based on the mean arterial pressure marker value includes: Determine the mean arterial pressure classification value corresponding to the mean arterial pressure marker value based on the preset mean arterial pressure-classification value mapping relationship;

The determination of the blood oxygen saturation classification value based on the blood oxygen saturation marker value includes:Determine the blood oxygen saturation classification value corresponding to the blood oxygen saturation marker value based on the preset blood oxygen saturation-classification value mapping relationship;

The determination of the lactate level classification value based on the lactate level marker value includes:Determine the lactate level classification value corresponding to the lactate level marker value based on the preset lactate level-classification value mapping relationship.

The further technical solution is that when the operating mode of the ECMO device is the preset venovenous (VV) ECMO mode:Determining the patient's ECMO operation score based on the blood oxygen saturation classification value, the lactate level classification value and the oxygen partial pressure classification value or the mean arterial pressure classification value includes:

Obtain the first physiological parameter-score mapping relationship between the preset oxygen partial pressure classification value, the blood oxygen saturation classification value, and the lactate level classification value and the ECMO operation score value;

Determine the patient's ECMO operation score value through the first physiological parameter-score mapping relationship based on the oxygen partial pressure classification value, the blood oxygen saturation classification value and the lactate level classification value.

Its further technical solution is that the first physiological parameter-score mapping relationship includes the following functional relationship: $S=A^2*B*C$, where S is the ECMO operation score value, A is the lactate level classification value, and B is the blood oxygen level. The saturation classification value and C are the oxygen partial pressure classification values.

Its further technical solution is that when the operating mode of the ECMO device is the preset venoarterial (VA) ECMO mode:Determining the patient's ECMO operation score based on the blood oxygen saturation classification value, the lactate level classification value and the oxygen partial pressure classification value or the mean arterial pressure classification value includes:

Obtain the preset second physiological parameter-score mapping relationship between the mean arterial pressure classification value, the blood oxygen saturation classification value, and the lactate level classification value and the ECMO operation score value;

Based on the mean arterial pressure classification value, the blood oxygen saturation classification value and the lactate level classification value, the patient's ECMO operation score is determined through the second physiological parameter-score mapping relationship.

Its further technical solution is that the second physiological parameter-score mapping relationship includes the following functional relationship: $S=A^2*B*D$, where S is the ECMO operation score value, A is the lactate level classification value, and B is the blood oxygen level. The saturation classification value and D are the mean arterial pressure classification values.

Its further technical solution is that the method also includes:

If the ECMO operation score satisfies $S \geq S1$, a reminder message is sent to increase the flow rate of ECMO equipment;

If the ECMO operation score value satisfies $S3 \leq S \leq S2$, a prompt message is issued to reduce the flow rate of the ECMO equipment;

If the ECMO operation score value satisfies $S < S3$, a prompt message is issued to remove the ECMO equipment;

Wherein, S is the ECMO operation score value, S1 is the first score threshold, S2 is the second score threshold, and S3 is the third score threshold.

Its further technical solution is that the method also includes:

Obtain the patient's physiological parameters collected by the monitoring equipment and ECMO equipment;
The patient's physiological parameters are stored in a time-aligned manner.

Its further technical solution is that the method also includes:

Obtain a training data set;
Based on the training data set, a pre-set initial classifier is trained through a preset machine learning algorithm to obtain a target classifier, which includes the oxygen partial pressure classifier and the mean arterial pressure classifier. , the blood oxygen saturation classifier, and the lactate level classifier.

Its further technical solution is that the method also includes:
Target data is filtered out from the physiological parameters, and the target data is displayed on a preset display device.
In a second aspect, examples of the present disclosure also provide a patient ECMO operation status assessment device, which includes a unit for executing the above method.
In a third aspect, examples of the present disclosure also provide a computer device, which includes a memory and a processor. A computer program is stored on the memory, and the above method is implemented when the processor executes the computer program.
In a fourth aspect, examples of the present disclosure also provide a computer-readable storage medium, the storage medium stores a computer program, and the computer program can implement the above method when executed by a processor.
Embodiments of the present disclosure provide a method, device, equipment and medium for evaluating the patient's ECMO operation status. wherein, the method comprises:Collect the patient's blood oxygen saturation data, lactate data, oxygen partial pressure data or mean arterial pressure data; based on the oxygen partial pressure data, obtain the oxygen partial pressure classification value through a pre-trained oxygen partial pressure classifier, or based on the mean arterial pressure data, Obtain the mean arterial pressure classification value through the pre-trained mean arterial pressure classifier; based on the blood oxygen saturation data, obtain the blood oxygen saturation classification value through the pre-trained blood oxygen saturation classifier; obtain the lactate level classification value based on the lactate data, determine the patient's ECMO operation through a pre-trained lactate level classifier based on the blood oxygen saturation classification value, the lactate level classification value, and the oxygen partial pressure classification value or the mean arterial pressure classification value. It can be seen that the patient's ECMO operation score can be accurately calculated through the above method, and the patient's recovery status can be intuitively determined through the ECMO operation score, which facilitates medical staff to make accurate judgments and reduces the decision-making time of medical staff.

## Brief Description of the Drawings

In order to more clearly illustrate the technical solutions of the examples of the present disclosure, the drawings needed to be used in the description of the examples will be briefly introduced below. The drawings in the following description are some examples of the present disclosure. For those in the field, ordinary technicians can also obtain other drawings based on these drawings without undue experimentation.

Figure 1 is a schematic flow chart of a method for evaluating a patient's ECMO operation status provided by an example of the present disclosure.
Figure 2 is a schematic diagram of the connection between the ECMO equipment and the monitoring equipment provided by the example of the present disclosure.
Figure 3 is a schematic block diagram of a patient ECMO operation status assessment device provided by an example of the present disclosure.
Figure 4 is a schematic block diagram of a computer device provided by an example of the present disclosure.

## Detailed Description

The technical solutions in the examples of the present disclosure will be clearly and completely described below with reference to the accompanying drawings in the examples of the present disclosure. Obviously, the described examples are part of the examples of the present disclosure, not all of them. Based on the examples of the present disclosure, all other examples obtained by those of ordinary skill in the art without any creative effort shall fall within the scope of protection of the present disclosure.

It is to be understood that, when used in this specification and the appended claims, the terms "includes" and "comprises" indicate the presence of described features, integers, steps, operations, elements and/or components but do not exclude the presence of one or the presence or addition of multiple other features, integers, steps, operations, elements, components and/or collections thereof.

It should also be understood that the terminology used in this description of the disclosure is for the purpose of describing particular examples only and is not intended to be limiting of the disclosure. As used in this specification and the appended claims, the singular forms "a", "an" and "the" are intended to include the plural forms unless the context clearly dictates otherwise.

It should also be further understood that the term "and/or" as used in this specification and the appended claims refers to and includes any and all possible combinations of one or more of the associated listed items.

As used in this specification and the appended claims, the term "if" may be interpreted as "when" or "once" or "in response to determining" or "in response to detecting" depending on the context. Similarly, the phrase "if determined" or "if [the described condition or event] is detected" may be interpreted, depending on the context, to mean "once determined" or "in response to determining" or "once the [described condition or event] is detected" or "in response to detection of [the described condition or event]".

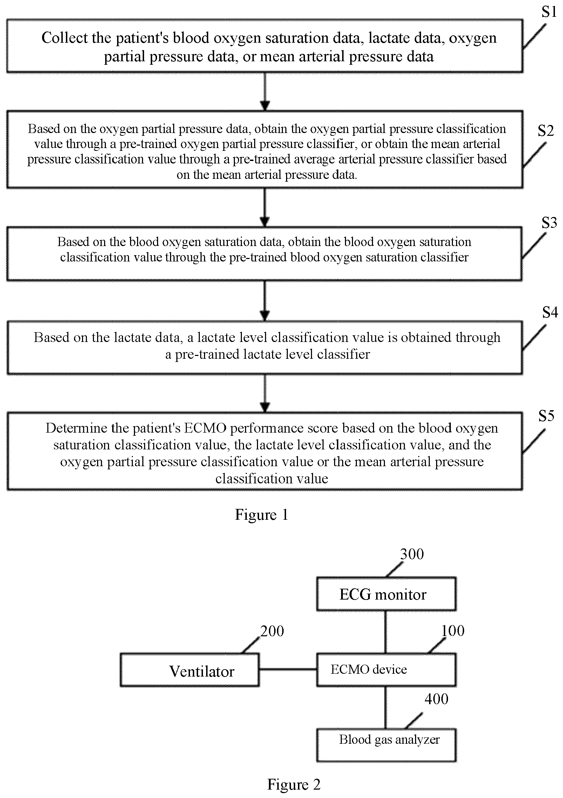

Please refer to Figure 1. An example of the present disclosure provides a method for evaluating a patient's ECMO operation status. The method includes the following steps:

Step S1 is to collect the patient's blood oxygen saturation data, lactate data, oxygen partial pressure data or mean arterial pressure data.

In specific implementation, the operating modes of extracorporeal membrane oxygenation (ECMO) equipment include venovenous (VV) ECMO mode and venoarterial (VA) ECMO mode. Specifically, ECMO is used in critically ill patients with severe lung and/or heart failure. Blood is drained from the venous system and oxygenated blood is pumped through a membrane oxygenator. There are two main circulation modes. For pulmonary support, blood is returned to the patient via veins (corresponding to VV ECMO mode), and for pulmonary/circulatory support, blood is returned to the patient via arteries (corresponding to VA ECMO mode).

In the VV ECMO mode, the patient's blood oxygen saturation data, lactate data, and oxygen partial pressure data are collected, that is, the patient's ECMO operation score is determined through the blood oxygen saturation data, lactate data, and oxygen partial pressure data. In an example of the present disclosure, blood oxygen saturation data, lactate data and oxygen partial pressure data are collected in no particular order, and the data collection processes of blood oxygen saturation data, lactate data and oxygen partial pressure data can be independent of each other.

In VA ECMO mode, the patient's blood oxygen saturation data, lactate data, and mean arterial pressure data are collected, that is, the patient's ECMO operation score is determined through the blood oxygen saturation data, lactate data, and mean arterial pressure data. In an example of the present disclosure, blood oxygen saturation data, lactate data and mean arterial pressure data are collected in no particular order, and the data collection processes of blood oxygen saturation data, lactate data and mean arterial pressure data can be independent of each other.

Specifically, the blood oxygen saturation data may be the patient's peripheral oxygen saturation ($SpO_2$) collected by a finger pulse oximeter, or the patient's arterial oxygen saturation ($SaO_2$) collected by a blood gas analyzer, which is not specifically limited by the present disclosure. It should be noted that $SpO_2$ is the transcutaneous arterial oxygen saturation measured by the distal circulation, and $SaO_2$ is the arterial oxygen saturation measured by arterial blood gas analysis.

Lactate data refers to the patient's blood lactate concentration (BLC) collected from the blood gas analyzer. Oxygen partial pressure data are also collected from the patient using a blood gas analyzer.

Mean arterial pressure data are collected from patients through an electrocardiogram monitor.

Step S2 is to, based on the oxygen partial pressure data, obtain the oxygen partial pressure classification value through a pre-trained oxygen partial pressure classifier, or based on the mean arterial pressure data, obtain the mean arterial pressure classification value through a pre-trained mean arterial pressure classifier.

In a specific implementation, the present disclosure pre-trains a target classifier, and the target classifier includes the oxygen partial pressure classifier, the mean arterial pressure classifier, the blood oxygen saturation classifier, and the lactate level classifier. The training process includes: obtaining a training data set; based on the training data set, training the pre-built initial classifier through a preset machine learning algorithm to obtain the target classifier.

Specifically, the target classifier is implemented using machine learning algorithms, which are not limited to nonlinear regression, classification algorithms, neural networks, support vector machines (SVM), and other models. The multiple target classifiers used in the present disclosure can be trained using the same or different machine learning algorithms, and are not specifically limited by the present disclosure.

In some embodiments, each target classifier is trained from the training data set. The training data set includes labeled data {(x, y)} of N training patients (i=1, ... N, wherein N is a positive integer). Among them, each training patient i is represented by a vector x and a label y of patient data (such as characteristic parameters of vital signs). The elements of the training patient data vector x are called "features" in this disclosure. The label y represents the marker value for training patient i. For example, at training time, the classifier's label y could be a binary value indicating whether training patient i was diagnosed. Alternatively, the label y can represent more information, for example the label yi used to train the mean arterial pressure classifier can be an integer value in the range between 0 and 100, where a value of 0-20 indicates the training patient's blood pressure flow index is low, the treatment effect is ineffective, or the person has died. A value of 90-100 indicates a training patient's hemodynamic index is good and meets expectations, and the ECMO can be weaned. An intermediate value (e.g., 21-89) indicates an uncertain state, which may require medical intervention in some situations. Continuous output can also be mapped into the range [0, 1], where 0 indicates that no anomaly is detected, and 1 indicates that an anomaly is detected and a warning needs to be issued.

The target classifier can also be trained to minimize the target function. For example, the form is:

$$\text{Objective function} = \sum(\text{observed value - theoretical value})^2$$

The observed value is the actual value in multiple groups of samples, and the theoretical value is the calculated value in the hypothetical fitting function. The objective function is also commonly referred to as the loss function in machine learning. The goal of the present disclosure is to obtain a model of the fitting function that minimizes the objective function and determine it using the least squares method. The sample is designed with multiple loss functions in the following form:

$$h_\Theta(x) = {}^\Theta 0\% + {}^\Theta_1 x_1 + {}^\Theta_2 x_2 + \ldots + {}^\Theta_n x_n., \bullet ({}^\Theta 0, {}^\Theta_1, {}^\Theta_2, \ldots, {}^\Theta_n)$$

where $h^\Theta(x)$ is the loss function, $\Theta_0, \Theta_1, \Theta_2, \ldots, \Theta n$, is the parameter of the loss function, X is the training data, including $X_1$, $X_2, \ldots, X_{no}$ is the optimal loss function obtained by training.

The classifier output trained by training samples has different output formats (such as binary, multi-classification, etc.) for each different prediction target in different classifiers.

In the example of the present disclosure, in the VV ECMO mode, based on the oxygen partial pressure data, the oxygen partial pressure classification value is obtained through a pre-trained oxygen partial pressure classifier.

In a specific implementation, the oxygen partial pressure data is input into the oxygen partial pressure classifier, and the oxygen partial pressure classification value is determined based on the result output by the oxygen partial pressure classifier.

For example, in one example, the above step S2 of "obtain the oxygen partial pressure classification value through a pre-trained oxygen partial pressure classifier based on the oxygen partial pressure data" specifically includes the following steps: inputting the oxygen partial pressure data into the oxygen partial pressure classifier; receiving the oxygen partial pressure marker value output by the oxygen partial pressure classifier; and determining the oxygen partial pressure classification based on the oxygen partial pressure marker value value.

In a specific implementation, the oxygen partial pressure classifier classifies the oxygen partial pressure data to obtain the corresponding oxygen partial pressure marker value. Afterwards, based on the preset oxygen partial pressure-classification value mapping relationship, the oxygen partial pressure classification value corresponding to the oxygen partial pressure marker value is determined. The mapping relationship between oxygen partial pressure and classification value can be set by those skilled in the art, and is not specifically limited by the present disclosure. For example, in one example, the mapping relationship between oxygen partial pressure and classification value is as shown in Table 1 below.

Table 1: Oxygen partial pressure - classification mapping table

| Oxygen partial pressure marker value | Oxygen partial pressure classification value |
| --- | --- |
| >100 | 1 |
| 80-100 | 3 |
| 70-80 | 5 |
| 60-70 | 7 |
| <60 | 9 |

In VA ECMO mode, based on the mean arterial pressure data, the mean arterial pressure classification value is obtained through a pre-trained mean arterial pressure classifier.

In a specific implementation, the mean arterial pressure data is input into the mean arterial pressure classifier, and the mean arterial pressure classification value is determined based on the result output by the mean arterial pressure classifier.

In one example, the above step S2 of "based on the mean arterial pressure data, obtaining the mean arterial pressure classification value through a pre-trained mean arterial pressure classifier" specifically includes the following steps: inputting the mean arterial pressure data into the mean arterial pressure classifier; receiving the mean arterial pressure flag value output by the mean arterial pressure classifier; and determining the mean arterial pressure classification based on the mean arterial pressure flag value value.

In a specific implementation, the mean arterial pressure classifier obtains the corresponding mean arterial pressure marker value by classifying the mean arterial pressure data. Afterwards, based on the preset mean arterial pressure-classification value mapping relationship, the mean arterial pressure classification value corresponding to the mean arterial pressure marker value is determined. The mapping relationship between the mean arterial pressure and the classification value can be set by those skilled in the art, and is not specifically limited by the present disclosure. For example, in one example, the mapping relationship between the mean arterial pressure and the classification value is as shown in Table 2.

Table 2: Mean Arterial Pressure - Categorical Value Mapping Table

| Mean arterial pressure marker value | Mean arterial pressure classification value |
|---|---|
| >75 | 1 |
| 70-75 | 3 |
| 65-70 | 5 |
| 55-65 | 7 |
| <55 | 9 |

Step S3 is to, based on the blood oxygen saturation data, obtain the blood oxygen saturation classification value through the pre-trained blood oxygen saturation classifier.

In a specific implementation, the blood oxygen saturation data is input into the blood oxygen saturation classifier, and the blood oxygen saturation classification value is determined based on the result output by the blood oxygen saturation classifier.

In one example, the above step S3 of "based on the blood oxygen saturation data, obtaining the blood oxygen saturation classification value through the pre-trained blood oxygen saturation classifier" specifically includes the following steps: inputting the blood oxygen saturation data into the blood oxygen saturation classifier; receiving the blood oxygen saturation marker value output by the blood oxygen saturation classifier; and determining the blood oxygen saturation marker value based on the blood oxygen saturation marker value and the blood oxygen saturation classification value.

In a specific implementation, the blood oxygen saturation classifier obtains the corresponding blood oxygen saturation marker value by classifying the blood oxygen saturation data. Afterwards, based on the preset blood oxygen saturation-classification value mapping relationship, the blood oxygen saturation classification value corresponding to the blood oxygen saturation marker value is determined. The mapping relationship between blood oxygen saturation and classification values can be set by those skilled in the art, and is not specifically limited by the present disclosure. For example, in one example, the mapping relationship between blood oxygen saturation and classification values is as detailed in Table 3 below.

Table 3: Blood oxygen saturation -classification value mapping relationship table

| Blood oxygen saturation marker value | Blood oxygen saturation classification value |
|---|---|
| 96-100 | 1 |
| 90-95 | 3 |
| 85-90 | 5 |
| 80-85 | 7 |
| <80 | 9 |

Step S4 is to, based on the lactate data, obtain lactate level classification values through a pre-trained lactate level classifier.

In a specific implementation, the lactic acid data is input into the lactic acid level classifier, and the lactic acid level classification value is determined according to the result output by the lactic acid level classifier.

In one example, the above step S4 of "based on the lactate data, obtain the lactate level classification value through the pre-trained lactate level classifier" specifically includes the following steps: inputting the lactate data into the lactate level classifier; receiving a lactate level marker value output by the lactate level classifier; and determining the lactate level classification value based on the lactate level marker value.

In a specific implementation, the lactate level classifier classifies the lactate data to obtain the corresponding lactate level marker value. Afterwards, based on the preset lactate level-classification value mapping relationship, the lactate level classification value corresponding to the lactate level marker value is determined. The mapping relationship between lactate level and classification value can be set by those skilled in the art, and is not specifically limited by the present disclosure. For example, in one example, the mapping relationship between lactate level and classification value is as shown in Table 4 below.

Table 4: Lactate level - classification value mapping relationship table

| Lactate level marker value | Lactate level classification value |
| --- | --- |
| <2 | 1 |
| 2-3 | 3 |
| 3-4 | 5 |
| 4-5 | 7 |
| >5 | 9 |

In an example of the present disclosure, the above-mentioned steps S2-S4 are executed in parallel instead of in sequential series.

It should be noted that the above-mentioned blood oxygen saturation marker value, lactate level marker value, oxygen partial pressure marker value and mean arterial pressure marker value are all data read on the machine, therefore, they do not contain unit information.

Step S5 is to determine the patient's ECMO performance score based on the blood oxygen saturation classification value, the lactate level classification value, and the oxygen partial pressure classification value or the mean arterial pressure classification value.

In a specific implementation, when the operation mode of the ECMO device is the preset VV ECMO mode, the patient's ECMO operation score is determined based on the blood oxygen saturation classification value, the lactate level classification value and the oxygen partial pressure classification value value.

In the VV ECMO mode, the patient's blood oxygen saturation data, lactate data, and oxygen partial pressure data are collected, and the corresponding blood oxygen saturation is further calculated through the blood oxygen saturation data, lactate data, and oxygen partial pressure data. Degree classification value, the lactate level classification value, and the oxygen partial pressure classification value are determined, and then the patient's ECMO performance score is determined based on the blood oxygen saturation classification value, the lactate level classification value, and the oxygen partial pressure classification value value.

Specifically, in one example, the above step S5 of "determine the patient's ECMO operation score based on the blood oxygen saturation classification value, the lactate level classification value, and the oxygen partial pressure classification value" specifically includes: obtaining the preset first physiological parameter-score mapping relationship between the preset oxygen partial pressure classification value, the blood oxygen saturation classification value, and the lactate level classification value and the ECMO operation score value; and based on the oxygen partial pressure, determining the ECMO operation score value of the patient through the first physiological parameter-score mapping relationship based on the blood oxygen saturation classification value, the lactate level classification value, and the blood oxygen partial pressure classification value.

In a specific implementation, the first physiological parameter-score mapping relationship can be set by those skilled in the art, and is not specifically limited by the present disclosure.

For example, in one example, the scoring rule table in VVECMO mode is as shown in Table 5.

Table 5: Scoring rule table in VV ECMO mode

| Lactate level marker value | Lactate level classification value | Blood oxygen saturation marker value | Blood oxygen saturation classification value | Oxygen partial pressure marker value | Oxygen partial pressure classification value |
|---|---|---|---|---|---|
| <2 | 1 | 96-100 | 1 | >100 | 1 |
| 2-3 | 3 | 90-95 | 3 | 80-100 | 3 |
| 3-4 | 5 | 85-90 | 5 | 70-80 | 5 |
| 4-5 | 7 | 80-85 | 7 | 60-70 | 7 |
| >5 | 9 | <80 | 9 | <60 | 9 |

Correspondingly, the first physiological parameter-score mapping relationship includes the following functional relationship:

$S=A^2*B*C$, where S is the ECMO performance score value, A is the lactate level classification value, B is the blood oxygen saturation classification value, and C is the oxygen partial pressure classification value.

For example, in an example where A is 1, B is 3, and C is 5, then S=(1*1) *3*5=15.

In an example where A is 1, B is 5, and C is 7, then S=(1*1*5*7=35.

In these examples, when the calculated ECMO performance score value is <=35, it is proved that the VV ECMO flow rate can meet the patient's support needs. The system can inform the operator through an alarm or flash prompt that the ECMO flow rate can be reduced by 0.5L/min. Approximately 1 hour after adjusting the flow rate, the ECMO performance score is calculated again. If the value increases, or is >35, then the ECMO flow rate is increased to its previous state. If the score is <=35, the current ECMO flow rate will be maintained, the score will be calculated again the next day. This ECMO performance score value calculation and ECMO flow rate adjustment process can then be repeated over the following days.

In some embodiments of the present disclosure, the patient's recovery status is intuitively determined through the ECMO operation score value.

Further, when the operating mode of the ECMO device is the preset VA ECMO mode, the patient's ECMO operation score is determined based on the blood oxygen saturation classification value, the lactate level classification value, and the mean arterial pressure classification value.

In the VA ECMO mode, the patient's blood oxygen saturation data, lactate data, and mean arterial pressure data are collected, and the corresponding blood oxygen saturation data are further calculated based on the blood oxygen saturation data, lactate data, mean arterial pressure data degree classification value, the lactate level classification value, and the mean arterial pressure classification value. Then the patient's ECMO performance score is determined based on the blood oxygen saturation classification value, the lactate level classification value, and the mean arterial pressure classification value value.

Specifically, in one example, the above step S5 of "determine the patient's ECMO operation score based on the blood oxygen saturation classification value, the lactate level classification value and the mean arterial pressure classification value" specifically includes: obtaining the preset second physiological parameter-score mapping relationship between the mean arterial pressure classification value, the blood oxygen saturation classification value, and the lactate level classification value; and determining the ECMO operation score value based on the mean arterial pressure classification value, the blood oxygen saturation classification value, and the lactate level classification value using the second physiological parameter-score mapping relationship.

In a specific implementation, the second physiological parameter-score mapping relationship can be set by those skilled in the art, and is not specifically limited by the present disclosure.

For example, in one example, the scoring rule table in VA ECMO mode is as shown in Table 6.

Table 6: Scoring rule table in VA ECMO mode

| Lactate level marker value | Lactate level classification value | Blood oxygen saturation marker value | Blood oxygen saturation classification value | Mean arterial pressure marker value | Mean arterial pressure classification value |
|---|---|---|---|---|---|
| <2 | 1 | 96-100 | 1 | >75 | 1 |
| 2-3 | 3 | 90-95 | 3 | 70-75 | 3 |

(continued)

| Lactate level marker value | Lactate level classification value | Blood oxygen saturation marker value | Blood oxygen saturation classification value | Mean arterial pressure marker value | Mean arterial pressure classification value |
|---|---|---|---|---|---|
| 3-4 | 5 | 85-90 | 5 | 65-70 | 5 |
| 4-5 | 7 | 80-85 | 7 | 55-65 | 7 |
| >5 | 9 | <80 | 9 | <55 | 9 |

Correspondingly, the second physiological parameter-score mapping relationship includes the following functional relationship:

S=A$^2$*B*D, where, SECMO operation score value, A is the lactate level classification value, B is the blood oxygen saturation classification value, and D is the mean arterial pressure classification value.

For example, in one example, A is 1, B is 1 and D is 5, then S =(1*1)*1*5 = 5.

In one example, A is 1, B is 3 and D is 7, then S = (1*1)*3*7 = 21.

In these examples, when the calculated ECMO performance score value is <=21, it is proved that the VA ECMO flow rate can meet the patient's support needs. The system can inform the operator through an alarm or flash prompt that the ECMO flow rate can be reduced by 0.5L/min. Approximately 1 hour after adjusting the flow rate, the ECMO performance score is calculated again. If the value increases, or is >21, then the ECMO flow rate is increased to its previous state. If the score is <=21, the current ECMO flow rate will be maintained, the score will be calculated again the next day. This ECMO performance score value calculation and ECMO flow rate adjustment process can then be repeated over the following days.

In the present disclosure, the patient's recovery status can be intuitively determined through the ECMO operation score value.

In a technical solution of the example of the present disclosure, the patient's blood oxygen saturation data, lactate data, and oxygen partial pressure data or mean arterial pressure data are collected. Based on the oxygen partial pressure data, the blood oxygen partial pressure classification value is obtained using a pre-trained oxygen partial pressure classifier. Based on the mean arterial pressure data, the mean arterial pressure classification value is obtained using a pre-trained mean arterial pressure classifier. Based on the blood oxygen saturation data, a blood oxygen saturation classification value is obtained using a pre-trained blood oxygen saturation classifier. Based on the lactate data, a lactate level classification value is obtained through a pre-trained lactate level classifier. Based on the the blood oxygen saturation classification value, the lactate level classification value, and the oxygen partial pressure classification value, and/or the mean arterial pressure classification value, the patient's ECMO performance score is determined. It can be seen that the patient's ECMO operation score can be accurately calculated through the above method. The patient's recovery status can be intuitively determined through the ECMO operation score, which facilitates medical staff to make accurate judgments and reduces the decision-making time of medical staff.

In one example, after determining the patient's ECMO functioning score, the method further includes: if the ECMO operation score value satisfies S≥S1, a prompt message is issued to increase the flow rate of the ECMO device; if the ECMO operation score value satisfies S3≤S≤S2, a prompt message is issued to reduce the flow rate of the ECMO device; and if the ECMO operation score satisfies S<S3, a prompt message to remove the ECMO device is issued; where S is the ECMO operation score, S1 is the first score threshold, S2 is the second score threshold, and S3 is the third score threshold.

In specific implementation, the first score threshold, the second score threshold and the third score threshold are all set by those skilled in the art, and are not specifically limited by the present disclosure. When the ECMO operation score value is greater than the preset first score threshold, it means that the patient's recovery is poor, so a prompt message to increase the flow rate of the ECMO equipment is issued, prompting the operator to appropriately increase the flow rate of the ECMO equipment. When the ECMO operation score value is less than the preset second score threshold, it indicates that the patient's recovery is acceptable, so a prompt message to reduce the flow rate of the ECMO equipment is issued, prompting the operator to appropriately reduce the flow rate of the ECMO equipment. When the ECMO operation score value is less than the preset third score threshold, it indicates that the patient's recovery is very good, so a prompt message to remove the ECMO device is issued to remind the operator that the patient has met the criteria for removing the ECMO device. It can be understood that the first score threshold is greater than the second score threshold, and the second score threshold is greater than the third score threshold.

Further, when the ECMO operation score value is between the first score threshold and the second score threshold, the flow rate of the ECMO device is kept unchanged, and the patient's condition is further observed.

In one example, in order to better display the patient's physical condition, the method further includes the following steps:

Step S2.1 (which can be a substep of step S2) is to obtain the patient's physiological parameters collected by the monitoring device and the ECMO device.

In a specific implementation, monitoring equipment includes ventilators, ECG monitors, and blood gas analyzers.

Generally, ventilators for severe cases are invasive, and relatively few are non-invasive. In the present disclosure, physiological parameters such as tidal volume, minute ventilation, partial pressure of oxygen, and air flow are extracted from the invasive ventilator.

The physiological parameters collected by the ECG monitor include blood systolic blood pressure, diastolic blood pressure, heart rate, respiratory rate, blood oxygen saturation, and mean arterial pressure.

The physiological parameters collected by the blood gas analyzer include blood oxygen saturation (referred to as blood oxygen), carbon dioxide content, electrolyte levels, lactic acid, oxygen partial pressure, and blood pH. In the present disclosure, the oxygen partial pressure data used to calculate the ECMO operation score value comes from the blood gas analyzer, and the oxygen partial pressure data collected by the ventilator is not used.

Physiological parameters collected by the ECMO device include blood oxygen saturation, extracorporeal blood flow, and ECMO device operating parameters.

Referring to Figure 2, the ECMO device 100 provides electronic medical record (EMR) access capabilities based on the HL7 standard protocol of the medical device industry. The ECMO device 100 serves as a server, and the ventilator 200, ECG monitor 300, and blood gas analyzer 400 serve as clients. They are connected to the same internal local area network through wired or wireless methods, so that the ECMO device 100 can collect data from the ventilator 200, and electrocardiogram (ECG) data from monitoring equipment such as the electrical monitor 300 and the blood gas analyzer 400.

Step S2.2 (which can be a substep of step S2) is to store physiological parameters of patient in a time-aligned manner.

In a specific implementation, the patient's physiological parameters are stored in a time-aligned manner. Specifically, the physiological parameters corresponding to the same time are stored correspondingly, so that the specific conditions of each physiological parameter of the user at the same time can be determined.

Step S2.3 (which can be a substep of step S2) is to filter the target data from the physiological parameters, and display the target data in a preset display device.

In a specific implementation, by analyzing the correlation of data, the target data is filtered out, and the target data is presented. The present disclosure does not present all the data because through the target data, it can be quickly judged whether the patient's condition changes during the operation of the ECMO equipment and whether the relevant treatment measures are processed accordingly according to the operation of the ECMO.

Specifically, choosing to display the rotation speed and blood flow of the ECMO device allows medical staff to evaluate whether the patient's treatment is stable, whether the currently set treatment dose has been adjusted, whether the change in blood flow is consistent with the change in rotation speed, and whether it is a predetermined change.

In an example, the display of heart rate is not selected for the ECG monitor. In VV ECMO mode, the cardiac function is normal and the patient's heart rate changes are not the core evaluation index. The respiratory rate display is not selected because in VV ECMO mode, the patient's lung function is replaced by VV ECMO. Thus, the reference of respiratory rate is of little significance. At the same time, because the patient is under sedation or mechanical ventilation, monitoring the respiratory rate is not as accurate as observing the ventilator.

Selecting the display of pre-pump pressure P1 can reflect changes in the patient's effective blood volume, predict whether there are changes in the patient's blood flow due to insufficient volume, and thereby predict the risk of ECMO conversion.

In some embodiments, selecting the changes in invasive blood pressure (e.g., arterial blood pressure (ABP)) is useful because it can reflect the patient's hemodynamic condition. The invasive blood pressure reflects the patient's condition changes after transfer and reflects whether ECMO support is effective. In VV ECMO mode, effective and stable support can correct the patient's hypoxia, so the patient can maintain hemodynamic stability. Changes in invasive blood pressure at different time points can reflect changes in the patient's condition. At the same time, changes in invasive blood pressure can indicate that changes in flow are due to changes in vascular resistance and cardiac systolic function. The diastolic blood pressure of invasive blood pressure indicates peripheral vascular resistance, the mean arterial pressure indicates the perfusion of the systemic circulation, and the difference between systolic blood pressure and diastolic blood pressure indicates the changes in cardiac contractility.

In some embodiments, $SpO_2$ (percutaneous arterial oxygen saturation) is selected because continuous monitoring of $SpO_2$ changes can reflect the supportive effect of ECMO and changes in the patient's lung function. In the VV ECMO mode, ECMO replaces lung function and supports treatment with the goal of improving the patient's oxygen supply and correcting hypoxia. The patient's $SpO_2$ reflects whether the patient's condition is relieved. At the same time, the trend of changes in $SpO_2$ can reflect changes in the patient's lung function.

In the blood gas results, the following data are mainly selected and presented in combination with ECMO and ECG monitor data.

Lac (lactate level): Because lactic acid is a metabolite of hypoxia in the body, it has sensitivity and specificity. The absolute value of lactate can indicate whether the patient is hypoxic and the degree of hypoxia The changes in lactate illustrate the therapeutic effect of ECMO. At the same time, combined with the changes in flow rate and invasive blood pressure, it can be evaluated whether the therapeutic dose of ECMO can achieve the therapeutic effect.

$PaO_2$ (arterial oxygen partial pressure): The arterial oxygen partial pressure can describe the gas diffusion function of the patient's lungs. During VV ECMO, the changing trend of arterial oxygen partial pressure in the regular blood gas results can explain the changes in the patient's lung condition.

In some embodiments, no other indicators were selected because these indicators are more related to other treatment methods, such as blood transfusion medication, kidney function, liver function, and the patient's nutritional metabolism. They are not direct references with significance for evaluating the effect of ECMO supportive treatment. Therefore, it is not included in the data presentation on the display to avoid disturbing medical professionals' evaluation and judgment of the effectiveness of ECMO treatment.

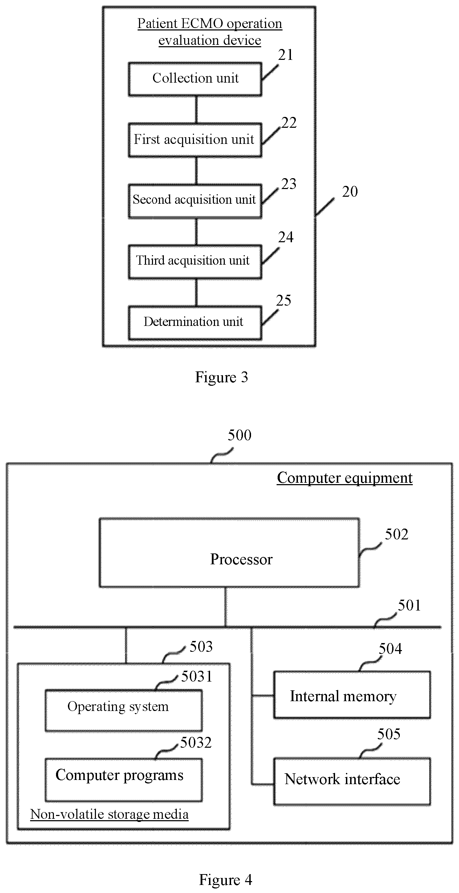

Referring to Figure 3, a schematic block diagram of a patient ECMO operation status evaluation device is shown. Corresponding to the above method for evaluating the patient's ECMO operation status, the present disclosure also provides a device for evaluating the patient's ECMO operation status. The patient's ECMO operation status evaluation device includes a unit for executing the above-mentioned patient's ECMO operation status evaluation method. The patient's ECMO operation status evaluation device can be configured in a desktop computer, a tablet computer, a laptop computer, and other terminals. Specifically, the patient's ECMO performance assessment device includes:

Collection unit 21, used to collect blood oxygen saturation data, lactate data, partial pressure of oxygen data, and/or mean arterial pressure data of the patient.

The first acquisition unit 22 is configured to acquire (or obtain, determine, or the like) an oxygen partial pressure classification value using a pre-trained oxygen partial pressure classifier based on the oxygen partial pressure data, and/or to aquire the mean arterial pressure classification value using a pre-trained mean arterial pressure classifier based on the mean arterial pressure data.

The second acquisition unit 23 is configured to acquire (or obtain, determine, or the like) the blood oxygen saturation classification value using the pre-trained blood oxygen saturation classifier based on the blood oxygen saturation data.

The third acquisition unit 24 is configured to acquire (or obtain, determine, or the like) a lactate level classification value using a pre-trained lactate level classifier based on the lactate data;

The determination unit 25 is configured to determine an ECMO performance score value of the patient based on the oxygen saturation classification value, the lactic acid level classification value, and the oxygen partial pressure classification value and/or the mean arterial pressure classification value.

A further technical solution is to obtain the oxygen partial pressure classification value through a pre-trained oxygen partial pressure classifier based on the oxygen partial pressure data, including: inputting the oxygen partial pressure data into the oxygen partial pressure classifier, and receiving the oxygen partial pressure marker value output by the oxygen partial pressure classifier; determining the oxygen partial pressure classification value based on the oxygen partial pressure marker value; based on the mean arterial pressure data, the mean arterial pressure classification value is obtained using a pre-trained mean arterial pressure classifier, including: inputting the mean arterial pressure data into the mean arterial pressure classifier, and receiving the mean arterial pressure marker value output by the mean arterial pressure classifier; determining the mean arterial pressure classification value based on the mean arterial pressure marker value; obtaining the blood oxygen saturation classification value using a pre-trained blood oxygen saturation classifier based on the blood oxygen saturation data, including: inputting the blood oxygen saturation data into the blood oxygen saturation classifier, and receiving the blood oxygen saturation marker value output by the blood oxygen saturation classifier; determining the blood oxygen saturation classification value based on the blood oxygen saturation marker value; based on the lactate data, the lactate level classification value is obtained through a pre-trained lactate level classifier, including: input the lactate data into the lactate level classifier, and receiving a lactate level marker value output by the lactate level classifier; and determine the lactate level classification value based on the lactate level marker value.

A further technical solution is to determine the oxygen partial pressure classification value based on the oxygen partial pressure marker value, including: determining the oxygen partial pressure classification value corresponding to the oxygen partial pressure marker value based on the preset oxygen partial pressure-classification value mapping relationship; wherein the determination of the mean arterial pressure classification value based on the mean arterial pressure marker value includes: determining the mean arterial pressure classification value corresponding to the mean arterial pressure marker value based on the preset mean arterial pressure-classification value mapping relationship; wherein the determining of the oxygen saturation classification value based on the oxygen saturation marker value comprises: determining the blood oxygen saturation classification value corresponding to the blood oxygen saturation marker value based on the preset blood oxygen saturation-classification value mapping relationship; wherein the determination of the lactate level classification value based on the lactate level marker value includes: determining the lactate level classification value corresponding to the lactate level marker value based on the preset lactate level-classification value mapping relationship.

The further technical solution is that when the operating mode of the ECMO device is the preset VV ECMO mode: the determining of the ECMO performance score value for the patient based on the oxygen saturation classification value, the lactate level classification value, and the partial pressure of oxygen classification value or the mean arterial pressure classification value, comprises: obtaining the first physiological parameter-score mapping relationship between the preset oxygen partial pressure classification value, the blood oxygen saturation classification value, and the lactate level classification value and the ECMO operation score value; determining the patient's ECMO operation score value through the first physiological parameter-score mapping relationship based on the oxygen partial pressure classification value, the blood oxygen saturation classification value and the lactate level classification value.

The further technical solution is that the first physiological parameter-score mapping relationship comprises the following functional relationship: $S=A^2*B*C$, where $S$ is the ECMO running score value, $A$ is the lactic acid level classification value, $B$ is the blood oxygen saturation classification value, and $C$ is the oxygen partial pressure classification value.

Its further technical solution is that when the operating mode of the ECMO device is the preset VA ECMO mode: determining the patient's ECMO operation score based on the blood oxygen saturation classification value, the lactate level classification value, and the oxygen partial pressure classification value and/or the mean arterial pressure classification value includes: acquiring a preset second physiological parameter-score mapping relationship between the mean arterial pressure classification value, the oxygen saturation classification value, the lactate level classification value, and the ECMO operation score value; nased on the mean arterial pressure classification value, the blood oxygen saturation classification value, and the lactate level classification value, the patient's ECMO operation score is determined through the second physiological parameter-score mapping relationship.

The further technical solution is that the second physiological parameter-score mapping relationship comprises the following functional relationship: $S=A^2*B*D$, where $S$ is the value of ECMO function score, $A$ is the classification value of lactic acid level, $B$ is the classification value of blood oxygen saturation, and $D$ is the classification value of mean arterial pressure.

Its further technical solution is that the method also includes: a first notification unit for sending a reminder to increase the flow rate of the ECMO device if the ECMO operation score satisfies $S{\geq}S1$; a second notification unit for sending a reminder message to reduce the flow rate of the ECMO device if the ECMO operation score value satisfies $S3{\leq}S{\leq}S2$; and a third notification unit is used to send a reminder message to remove the ECMO equipment if the ECMO operation score value satisfies $S<S3$; wherein, $S$ is the ECMO running score value, $S1$ is the first score threshold, $S2$ is the second score threshold, and $S3$ is the third score threshold.

The further technical solution is that the patient's ECMO performance evaluation device further comprises: a fourth acquiring unit for acquiring the monitoring device and the biological parameters of the patient acquired by the ECMO device; and a storage unit for storing physiological parameters of the patient in a time-aligned manner.

The further technical solution is that the patient's ECMO operation evaluation device further comprises: a fifth acquisition unit for acquiring a training data set; and a training unit for training a pre-built initial classifier based on the training data set by a pre-set machine learning algorithm to obtain a target classifier comprising the oxygen partial pressure classifier, the mean arterial pressure classifier, the oxygen saturation classifier, and the lactate level classifier.

The further technical solution is that the patient's ECMO performance evaluation device further comprises: a display unit for filtering target data from the physiological parameters, and displaying the target data in a preset display device.

It should be noted that those skilled in the art can clearly understand that the specific implementation process of the aforementioned patient ECMO operation evaluation device and each unit can be referred to the corresponding description in the above method example. For the sake of convenience and brevity of description, it will not be repeated here.

The aforementioned patient ECMO operation evaluation device may be implemented in the form of a computer program, which may be run on a computer device as shown in Figure 4.

Please refer to Figure 4, which is a schematic block diagram of a computer device 500 provided by an example of the present application. The computer device 500 may be a terminal or a server, where the terminal may be an electronic device with communication functions such as a smartphone, a tablet computer, a notebook computer, a desktop computer, a personal digital assistant, a wearable device, etc. The server can be an independent server or a server cluster composed of multiple servers.

The computer device 500 includes a processor 502, a memory, and a network interface 505 connected through a system bus 501, wherein the memory may include a non-volatile storage medium 503 and an internal memory 504.

The non-volatile storage medium 503 can store an operating system 5031 and computer programs 5032. One or more of the computer programs 5032 are configured to evaluate a patient's ECMO operation status. When such a computer program 5032 is executed, it causes the processor 502 to execute a method for evaluating the patient's ECMO operation status.

The processor 502 is used to provide computing and control capabilities to support the operation of the entire computer device 500.

The internal memory 504 provides an environment for the execution of the computer program 5032 in the non-volatile storage medium 503. When the computer program 5032 is executed by the processor 502, it can cause the processor 502 to execute a method for evaluating the patient's ECMO operation status.

The network interface 505 is used for network communication with other devices. Those skilled in the art can understand that the above structure is only a block diagram of a partial structure related to the solution of the present application, and does not constitute a limitation on the computer device 500 to which the solution of the present application is applied. The specific computer device 500 may include: More or fewer parts are shown, or certain parts are combined, or have different parts arrangements.

The processor 502 is used to run the computer program 5032 stored in the memory to implement the steps of a patient ECMO operation status assessment method as provided in any of the above method examples.

It should be understood that in the example of the present application, the processor 502 may be a central processing unit (Central Processing Unit, CPU). The processor 502 may also be other general-purpose processors, digital signal processors (Digital Signal Processor, DSP), Application Specific Integrated Circuit (ASIC), Field-Programmable Gate Array (FPGA) or other programmable logic devices, discrete gate or transistor logic devices, discrete hardware components, etc. Among them, the general processor may be a microprocessor or the processor may be any conventional processor, etc.

Those of ordinary skill in the art can understand that all or part of the processes in the methods of implementing the above examples can be completed by instructing relevant hardware through a computer program. The computer program can be stored in a storage medium, which is a computer-readable storage medium. The computer program is executed by at least one processor in the computer system to implement the process steps of the examples of the above method.

Accordingly, the present disclosure also provides a storage medium. The storage medium may be a computer-readable storage medium. The storage medium stores the computer program. When the computer program is executed by the processor, the computer program causes the processor to execute the steps of a patient ECMO operating status assessment method as provided in any of the above method examples.

The storage medium is a physical, non-transient storage medium, such as a USB flash drive, a mobile hard disk, a read-only memory (ROM), a magnetic disk or an optical disk, which can store program codes. The computer-readable storage medium may be non-volatile or volatile.

Those of ordinary skill in the art can realize that the units and algorithm steps of each example described in conjunction with the examples disclosed herein can be implemented with electronic hardware, computer software, or a combination of both. In order to clearly illustrate the relationship between hardware and software, Interchangeability, in the above description, the composition and steps of each example have been generally described according to function. Whether these functions are performed in hardware or software depends on the specific application and design constraints of the technical solution. Skilled technicians may use different methods to implement the described functions for each specific application, but such implementations should not be considered to be beyond the scope of the present disclosure.

In the several examples provided by the present disclosure, it should be understood that the disclosed devices and methods can be implemented in other ways. For example, the device examples described above are merely illustrative. For example, the division of each unit is only a logical function division, and there may be other division methods in actual implementation. For example, multiple units or components may be combined or integrated into another system, or some features may be omitted, or not implemented.

The steps in the methods of the examples of the present disclosure can be sequence adjusted, combined, and deleted according to actual needs. The units in the device of the example of the present disclosure can be merged, divided and deleted according to actual needs. In addition, each functional unit in various examples of the present disclosure can be integrated into one processing unit, or each unit can exist physically alone, or two or more units can be integrated into one unit.

If the integrated unit is implemented in the form of a software functional unit and sold or used as an independent product, it can be stored in a storage medium. Based on this understanding, the technical solution of the present disclosure essentially contributes to the existing technology, or all or part of the technical solution can be embodied in the form of a software product, and the computer software product is stored in a storage medium, including several instructions to cause a computer device (which can be a personal computer, terminal, or network device, etc.) to execute all or part of the steps of the method described in various examples of the present disclosure.

In the above examples, the description of each example has its own emphasis. For parts that are not described in detail in a certain example, please refer to the relevant descriptions of other examples.

Obviously, those skilled in the art can make various changes and modifications to the present disclosure without departing from the spirit and scope of the present disclosure. In this way, as long as these modifications and variations of the present disclosure fall within the scope of the claims of the present disclosure and equivalent technologies, the present disclosure is also intended to include these modifications and variations.

The above are only specific embodiments of the present disclosure, but the protection scope of the present disclosure is not limited thereto. Any person familiar with the technical field can easily think of various equivalent methods within the technical scope disclosed in the present disclosure. Modifications or substitutions, these modifications or substitutions should be covered by the protection scope of the present disclosure. Therefore, the scope of protection of the present disclosure shall be subject to the protection scope of the claims.

**Claims**

1.  A method for evaluating the extracorporeal membrane oxygenation (ECMO) operation status of patients, the method comprising:

    collecting a patient's blood oxygen saturation data, lactate data, oxygen partial pressure data, or mean arterial pressure data:
    obtaining the oxygen partial pressure classification value through a pre-trained oxygen partial pressure classifier based on the oxygen partial pressure data, or obtaining the mean arterial pressure classification value through a pre-trained mean arterial pressure classifier based on the mean arterial pressure data;
    obtaining the blood oxygen saturation classification value through a pre-trained blood oxygen saturation classifier based on the blood oxygen saturation data;
    obtaining a lactate level classification value through a pre-trained lactate level classifier based on the lactate data; and
    determining the patient's ECMO performance score based on the blood oxygen saturation classification value, the lactate level classification value, and the oxygen partial pressure classification value or the mean arterial pressure classification value.

2.  The method of claim 1, wherein based on the oxygen partial pressure data, the partial pressure of oxygen classification value is obtained through a pre-trained oxygen partial pressure classifier, the method further including:

    inputing the oxygen partial pressure data into the oxygen partial pressure classifier, and receiving the oxygen partial pressure marker value output by the oxygen partial pressure classifier;
    determining the oxygen partial pressure classification value based on the oxygen partial pressure marker value;
    obtaining the mean arterial pressure classification value through a pre-trained mean arterial pressure classifier, based on the mean arterial pressure data, including:
    inputting the mean arterial pressure data into the mean arterial pressure classifier, and receiving the mean arterial pressure marker value output by the mean arterial pressure classifier;
    determining the mean arterial pressure classification value based on the mean arterial pressure marker value;
    obtaining the blood oxygen saturation classification value through a pre-trained blood oxygen saturation classifier based on the blood oxygen saturation data, including:
    inputting the blood oxygen saturation data into the blood oxygen saturation classifier, and receiving the blood oxygen saturation marker value output by the blood oxygen saturation classifier;
    determining the blood oxygen saturation classification value based on the blood oxygen saturation marker value; and
    obtaining the lactate level classification value through a pre-trained lactate level classifier based on the lactate data, including:
    inputting the lactate data into the lactate level classifier, and receiving a lactate level marker value output by the lactate level classifier; and
    determining the lactate level classification value based on the lactate level marker value.

3.  The method of claim 2, wherein:

    determining the oxygen partial pressure classification value based on the oxygen partial pressure marker value includes determining the oxygen partial pressure classification value corresponding to the oxygen partial pressure marker value based on the preset oxygen partial pressure-classification value mapping relationship;
    determining the mean arterial pressure classification value based on the mean arterial pressure marker value includes determining the mean arterial pressure classification value corresponding to the mean arterial pressure marker value based on the preset mean arterial pressure-classification value mapping relationship;

determining the blood oxygen saturation classification value based on the blood oxygen saturation marker value includes determining the blood oxygen saturation classification value corresponding to the blood oxygen saturation marker value based on the preset blood oxygen saturation-classification value mapping relationship; and

determining the lactate level classification value based on the lactate level marker value includes determining the lactate level classification value corresponding to the lactate level marker value based on the preset lactate level-classification value mapping relationship.

4. The method of claim 1, wherein when the operation mode of the ECMO device is a preset venovenous (VV) ECMO mode, wherein determining the patient's ECMO operation score based on the blood oxygen saturation classification value, the lactate level classification value, and the oxygen partial pressure classification value or the mean arterial pressure classification value includes:

   obtaining the first physiological parameter-score mapping relationship between the preset oxygen partial pressure classification value, the blood oxygen saturation classification value, and the lactate level classification value and the ECMO operation score value; and

   determining the patient's ECMO operation score value through the first physiological parameter-score mapping relationship based on the oxygen partial pressure classification value, the blood oxygen saturation classification value and the lactate level classification value.

5. The method of claim 4, wherein the first physiological parameter-score mapping relationship includes the following functional relationship: $S=A^2*B*C$, wherein S is the ECMO operation score value, A is the lactate level classification value, B is the blood oxygen saturation classification value, and C is the oxygen partial pressure classification value.

6. The method of claim 1, wherein when the operating mode of the ECMO device is a preset venoarterial (VA) ECMO mode, the classification based on the blood oxygen saturation classification value and the lactate level value and the oxygen partial pressure classification value or the mean arterial pressure classification value to determine the patient's ECMO performance score value, the method further including:

   obtaining the second physiological parameter-score mapping relationship between the preset mean arterial pressure classification value, the blood oxygen saturation classification value, and the lactate level classification value and the ECMO operation score value; and

   determining the patient's ECMO operation score value through the second physiological parameter-score mapping relationship based on the mean arterial pressure classification value, the blood oxygen saturation classification value and the lactate level classification value.

7. The method of claim 6, wherein the second physiological parameter-score mapping relationship includes the following functional relationship: $S=A^2*B*D$, wherein S is the ECMO operation score value, A is the lactate level classification value, B is the blood oxygen saturation classification value, and D is the mean arterial pressure classification value.

8. The method of claim 1, further comprising:

   sending a reminder message to increase the flow rate of ECMO device in response to the ECMO operation score satisfying a relationship of $S \geq S1$;

   issuing a prompt message to reduce the flow rate of the ECMO device in response to the ECMO operation score value satisfying a relationship of $S3 \leq S \leq S2$; and

   sending a prompt message to remove the ECMO device in response to the ECMO running score satisfying a relationship of $S<S3$;

   wherein, S is the ECMO running score value, S1 is the first score threshold, S2 is the second score threshold, and S3 is the third score threshold.

9. The method of claim 1, further comprising:

   obtaining the patient's physiological parameters collected by the monitoring equipment and ECMO equipment; and

storing the patient's physiological parameters in a time-aligned manner.

10. The method of claim 9, further comprising:

filtering target data out from the physiological parameters; and
displaying the target data on a preset display device.

11. The method of claim 1, **characterized in that** the method further comprises:
obtaining a training data set; and
training a pre-set initial classifier is trained through a preset machine learning algorithm to obtain a target classifier based on the training data set, which includes the oxygen partial pressure classifier and the mean arterial pressure classifier, the blood oxygen saturation classifier, and the lactate level classifier.

12. A patient ECMO operation status assessment device, **characterized by** comprising an ECMO device that is configured to execute the method of claim 1.

13. A computer device for evaluating the extracorporeal membrane oxygenation (ECMO) operation status of patients comprising:

a processor;
a memory connected to the processor, the memory including a computer program that, when executed by the processor, causes the processor to:
collect a patient's blood oxygen saturation data, lactate data, oxygen partial pressure data, or mean arterial pressure data:
obtain the oxygen partial pressure classification value through a pre-trained oxygen partial pressure classifier based on the oxygen partial pressure data, or obtain the mean arterial pressure classification value through a pre-trained mean arterial pressure classifier based on the mean arterial pressure data;
obtain the blood oxygen saturation classification value through a pre-trained blood oxygen saturation classifier based on the blood oxygen saturation data;
obtain a lactate level classification value through a pre-trained lactate level classifier based on the lactate data; and
determine the patient's ECMO performance score based on the blood oxygen saturation classification value, the lactate level classification value, and the oxygen partial pressure classification value or the mean arterial pressure classification value.

14. A computer-readable storage medium that stores a computer program for evaluating an extracorporeal membrane oxygenation (ECMO) operation status of patients, the computer program including instructions to:

collect a patient's blood oxygen saturation data, lactate data, oxygen partial pressure data, or mean arterial pressure data:
obtain the oxygen partial pressure classification value through a pre-trained oxygen partial pressure classifier based on the oxygen partial pressure data, or obtain the mean arterial pressure classification value through a pre-trained mean arterial pressure classifier based on the mean arterial pressure data;
obtain the blood oxygen saturation classification value through a pre-trained blood oxygen saturation classifier based on the blood oxygen saturation data;
obtain a lactate level classification value through a pre-trained lactate level classifier based on the lactate data; and
determine the patient's ECMO performance score based on the blood oxygen saturation classification value, the lactate level classification value, and the oxygen partial pressure classification value or the mean arterial pressure classification value

Collect the patient's blood oxygen saturation data, lactate data, oxygen partial pressure data, or mean arterial pressure data

S1

Based on the oxygen partial pressure data, obtain the oxygen partial pressure classification value through a pre-trained oxygen partial pressure classifier, or obtain the mean arterial pressure classification value through a pre-trained average arterial pressure classifier based on the mean arterial pressure data.

S2

Based on the blood oxygen saturation data, obtain the blood oxygen saturation classification value through the pre-trained blood oxygen saturation classifier

S3

Based on the lactate data, a lactate level classification value is obtained through a pre-trained lactate level classifier

S4

Determine the patient's ECMO performance score based on the blood oxygen saturation classification value, the lactate level classification value, and the oxygen partial pressure classification value or the mean arterial pressure classification value

S5

Figure 1

ECG monitor — 300

Ventilator — 200

ECMO device — 100

Blood gas analyzer — 400

Figure 2

Patient ECMO operation
evaluation device

21
Collection unit

22
First acquisition unit

23
Second acquisition unit

24
Third acquisition unit

25
Determination unit

20

Figure 3

500

Computer equipment

502
Processor

501

503
5031
Operating system

5032
Computer programs

Non-volatile storage media

504
Internal memory

505
Network interface

Figure 4

Europäisches
Patentamt

European
Patent Office

Office européen
des brevets

# EUROPEAN SEARCH REPORT

**Application Number**

EP 25 15 2263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MESSAÏ ELMI ET AL: "A new formula for determining arterial oxygen saturation during venovenous extracorporeal oxygenation", INTENSIVE CARE MEDICINE, SPRINGER BERLIN HEIDELBERG, BERLIN/HEIDELBERG, vol. 39, no. 2, 5 December 2012 (2012-12-05), pages 327-334, XP038160530, ISSN: 0342-4642, DOI: 10.1007/S00134-012-2756-0 [retrieved on 2012-12-05] * columns 1-2, paragraph 328; figures 1,2; tables 1,2 * * page 330, columns 1-2 * | 1-14 | INV. G16H50/20 |
| A | HIDESHI ITOH ET AL: "Effect of the Pulsatile Extracorporeal Membrane Oxygenation on Hemodynamic Energy and Systemic Microcirculation in a Piglet Model of Acute Cardiac Failure", ARTIFICIAL ORGANS, BLACKWELL SCIENTIFIC PUBLICATIONS, INC., BOSTON, US, vol. 40, no. 1, 3 November 2015 (2015-11-03), pages 19-26, XP071484579, ISSN: 0160-564X, DOI: 10.1111/AOR.12588 * the whole document * | 1-14 | |
| Y | LUCA GIORDANO ET AL: "Predictive models in extracorporeal membrane oxygenation (ECMO): a systematic review", SYSTEMATIC REVIEWS, BIOMED CENTRAL LTD, LONDON, UK, vol. 12, no. 1, 15 March 2023 (2023-03-15) , pages 1-12, XP021315770, DOI: 10.1186/S13643-023-02211-7 * page 9, column 1 * | 1-14 | |

**TECHNICAL FIELDS SEARCHED (IPC)**

G16H

-/--

The present search report has been drawn up for all claims

| Place of search | Date of completion of the search | Examiner |
|---|---|---|
| Munich | 2 June 2025 | Laub, Christoph |

EPO FORM 1503 03.82 (P04C01)

page 1 of 2

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RIVERS JON ET AL: "Extracorporeal membrane oxygenation for the treatment of massive pulmonary embolism. An analysis of the ELSO database", RESUSCITATION, ELSEVIER, IE, vol. 191, 23 August 2023 (2023-08-23), XP087405377, ISSN: 0300-9572, DOI: 10.1016/J.RESUSCITATION.2023.109940 [retrieved on 2023-08-23] * the whole document * | 1-14 | |
| A | WO 2020/208258 A1 (UNIV BERN [CH]) 15 October 2020 (2020-10-15) * the whole document * | 1-14 | |

**TECHNICAL FIELDS SEARCHED (IPC)**

The present search report has been drawn up for all claims

| Place of search | Date of completion of the search | Examiner |
|---|---|---|
| Munich | 2 June 2025 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

        .........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 2263

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2025

| Patent document cited in search report | Publication date | Patent family member(s) | Publication date |
|---|---|---|---|
| WO 2020208258 A1 | 15-10-2020 | NONE | |

For more details about this annex : see Official Journal of the European Patent Office, No. 12/82

EPO FORM P0459